(19) Europäisches Patentamt
European Patent Office
Office européen des brevets

(11) Veröffentlichungsnummer: **0 032 254**
**B1**

# (12) EUROPÄISCHE PATENTSCHRIFT

(45) Veröffentlichungstag der Patentschrift: **16.05.84**

(51) Int. Cl.³: **C 07 C 121/50,** C 07 C 69/76 // D06L3/12

(21) Anmeldenummer: **80108265.2**

(22) Anmeldetag: **31.12.80**

## (54) Verfahren zur Herstellung von Bis-styrylbenzolen.

(30) Priorität: **12.01.80 DE 3001065**

(43) Veröffentlichungstag der Anmeldung: **22.07.81 Patentblatt 81/29**

(45) Bekanntmachung des Hinweises auf die Patenterteilung: **16.05.84 Patentblatt 84/20**

(84) Benannte Vertragsstaaten: **CH DE FR GB IT LI**

(56) Entgegenhaltungen:
**EP - A - 0 023 027**
**EP - A - 0 030 917**
**GB - A - 920 988**

(73) Patentinhaber: **BASF Aktiengesellschaft, Carl-Bosch-Strasse 38, D-6700 Ludwigshafen (DE)**

(72) Erfinder: **Seybold, Guenther, Dr., Otto-Dill-Strasse 32, D-6700 Ludwigshafen (DE)**

EP 0 032 254 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Bis-styrylbenzolen

Die Erfindung betrifft ein Verfahren zur Herstellung von unsymmetrischen Verbindungen oder Verbindungsgemischen der allgemeinen Formel

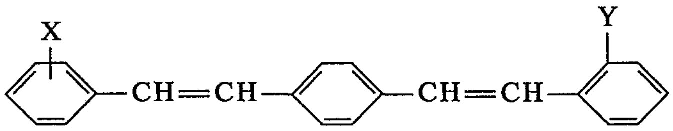
(I)

in der
X und Y unabhängig voneinander Wasserstoff, Fluor, Chlor, Cyan, $C_1$- bis $C_{10}$-Alkoxycarbonyl, gegebenenfalls substituiertes Carbamoyl oder Sulfamoyl, Sulfonsäurearylester, $C_1$- bis $C_{10}$-Alkylsulfonyl oder Phenylsulfonyl bedeuten, das dadurch gekennzeichnet ist, daß man Terephthalaldehyd mit Verbindungen der allgemeinen Formeln IIa und IIb

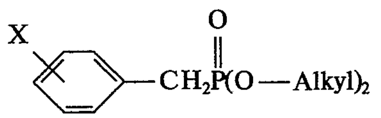
(IIa)

und

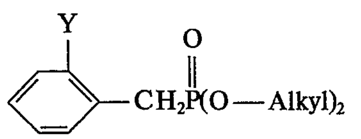
(IIb)

nacheinander in einem Ester als Lösungsmittel mit Alkali umsetzt, in dem das Monokondensationsprodukt ausfällt; X und Y haben dabei die angegebene Bedeutung und »Alkyl« ist $C_1$- bis $C_4$-Alkyl.

Einzelne Reste X und Y sind neben den bereits genannten beispielsweise:

$COOCH_3$  $COOC_2H_5$  $COOC_3H_7$  $COOC_4H_9$  $COOC_6H_{13}$  $COOC_8H_{17}$

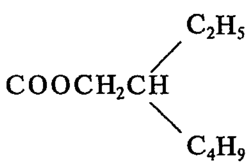  $COOC_{10}H_{21}$  $COOC_5H_{11}$  $CONHCH_3$  $CONHC_2H_5$

$CONHC_3H_7$  $CONHC_4H_9$  $CONHC_6H_{13}$  $CONHC_8H_{17}$  $CON(CH_3)_2$

$CON(C_2H_5)_2$  $CON(C_3H_7)_2$  $CON(C_4H_9)_2$  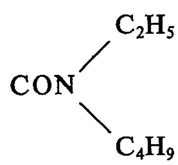  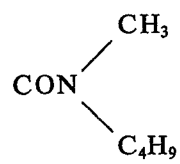

$CONHC_2H_4OH$  $CON(C_2H_4OH)_2$  $CONHC_2H_4OCH_3$  $CON(C_2H_4OCH_3)_2$

$CON(C_2H_4OC_4H_9)_2$  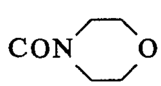  die entsprechenden Sulfamoylreste  $SO_2OC_6H_5$

$SO_2OC_6H_4CH_3$  $CH_3SO_2$  $SO_2C_2H_5$  $SO_2C_4H_9$  $SO_2C_6H_{13}$  oder  $SO_2C_8H_{17}$

Für die Umsetzung geeignete Lösungsmittel sind Ester, wie Äthylacetat, n- oder i-Butylacetat, Methylglykolacetat oder n- oder i-Propylacetat.

Bevorzugte Lösungsmittel sind:

Äthylacetat, n- oder i-Butylacetat oder Methylglykolacetat.

Zweckmäßigerweise wird das erfindungsgemäße Verfahren so durchgeführt, daß man den Terephthalaldehyd im Lösungsmittel auflöst und dann zunächst die Verbindung der Formel IIa und die zur Kondensation notwendige Menge Alkali langsam zusetzt. Nach Abschluß der Umsetzung, die sich z. B. dünnschichtchromatographisch oder gaschromatographisch verfolgen läßt, wird dann die Verbindung der Formel IIb und die dafür notwendige Alkalimenge zugegeben. Zweckmäßige Umsetzungstemperaturen liegen im Bereich von 30 bis 50° C, die Umsetzungsdauer beträgt in der Regel insgesamt 6 bis 12 Stunden.

Als Alkali sind insbesondere Alkalialkoholate wie Natrium- oder Kaliummethylat, -äthylat oder -butylat geeignet.

Einzelheiten des erfindungsgemäßen Verfahrens können den Beispielen entnommen werden, in denen sich Angaben über Teile und Prozente, sofern nicht anders vermerkt, auf das Gewicht beziehen.

Gegenüber dem in der GB-A-920 988 beschriebenen Verfahren hat das erfindungsgemäße Verfahren den Vorteil, daß man Verbindungen und Verbindungsgemische der allgemeinen Formel I herstellen kann, die keine p,p'-Isomeren (bezogen auf X und Y) enthalten. Die erfindungsgemäß hergestellten Verbindungen und Verbindungsgemische sind als optische Aufheller geeignet und zeichnen sich durch ein besonders günstiges Fixierverhalten und hohe Ausgiebigkeit aus.

Bevorzugt sind Gemische von Dicyanverbindungen, insbesondere von o,m'-Dicyan-bis-styrylbenzol und o,p'-Dicyan-bis-styrylbenzol, z. B. das Gemisch des Beispiels 14.

Beispiel 1

In eine Mischung von 146 Teilen Terephthalaldehyd (93%ig), 275 Teilen o-Cyanbenzylphosphonsäurediäthylester (93%ig) und 1200 Teilen Methylglykolacetat läßt man unter heftigem Rühren bei 30—35° C innerhalb von 4 Stunden 200 Teile Natriummethylatlösung (30%ig) eintropfen. Es wird 4 Stunden bei 30—35° C nachgerührt und anschließend werden 300 Teile p-Cyanphosphonester (85%ig) zugegeben. Bei 45 bis 50° C läßt man innerhalb von 4 Stunden 306 Teile Natriummethylatlösung 30%ig zutropfen. Es wird 5 Stunden bei 45—50° C nachgerührt, man kühlt auf Raumtemperatur ab, saugt das Produkt ab und wäscht gründlich mit Methanol nach.

Ausbeute: 260 Teile grünlich gelbe Kristalle (Schmp. 190—192° C) der Formel

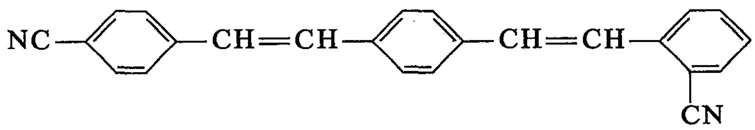

Beispiel 2

Es wurde wie in Beispiel 1 verfahren, jedoch wurden statt 1200 Teilen Methylglykolacetat 1100 Teile Äthylacetat verwendet.

Ausbeute: wie in Beispiel 1

Beispiel 3

Wie in Beispiel 1, als Lösungsmittel wurde Isobutylacetat verwendet.

Ausbeute: 78%

Beispiele 4—13

Es wurde analog dem Beispiel 1 gearbeitet, jedoch kamen folgende Lösungsmittel und Phosphonester zum Einsatz:

| Bei-spiel | Lösungs-mittel | Phosphonester IIa | Phosphonester IIb | Produkt |
|---|---|---|---|---|
| 4 | Äthyl-acetat | 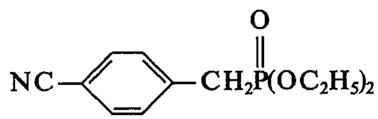 | 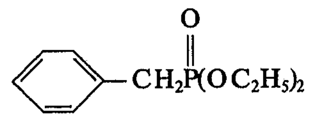 | 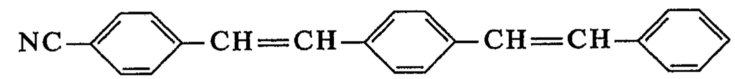 |
| 5 | desgl. | 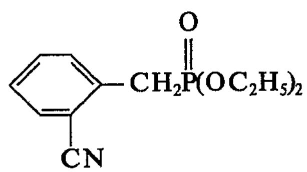 | 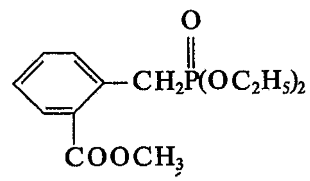 | 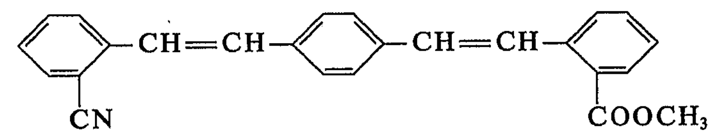 |
| 6 | Butyl-acetat | 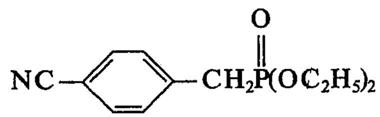 | 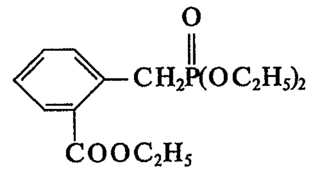 | 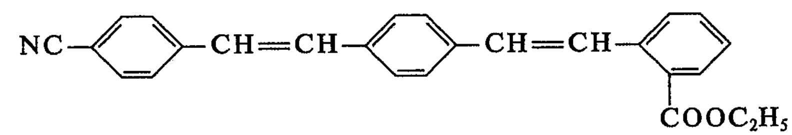 |
| 7 | desgl. | desgl. | 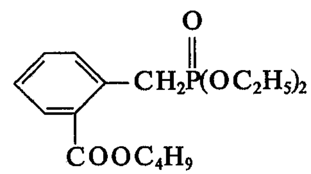 | 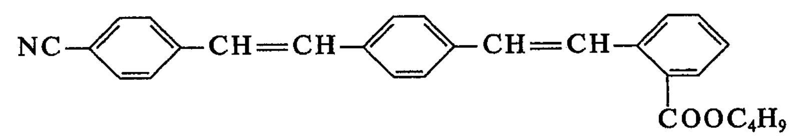 |
| 8 | Methyl-glykol-acetat | 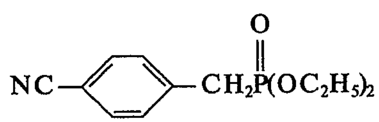 | 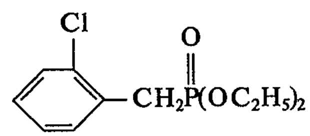 | 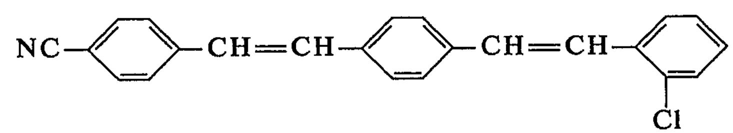 |
| 9 | desgl. | desgl. | 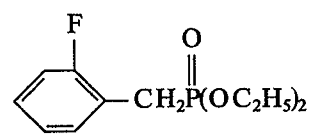 | 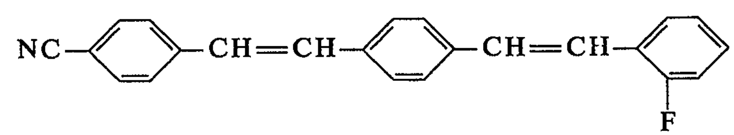 |

Fortsetzung

| Bei-spiel | Lösungs-mittel | Phosphonester IIa | Phosphonester IIb | Produkt |
|---|---|---|---|---|
| 10 | Äthyl-glykol-acetat | 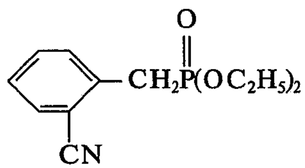 | 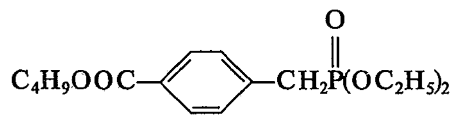 | 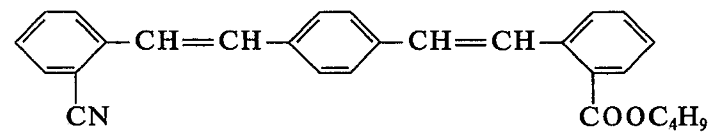 |
| 11 | desgl. | 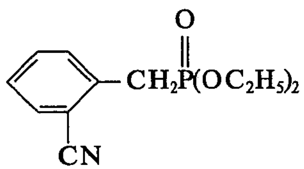 | 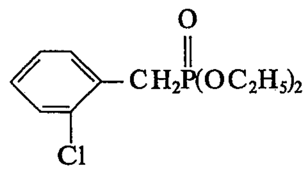 | 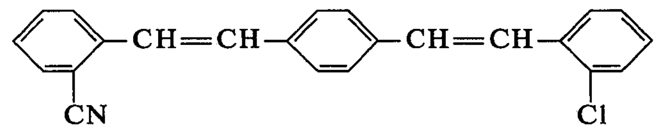 |
| 12 | desgl. | desgl. | 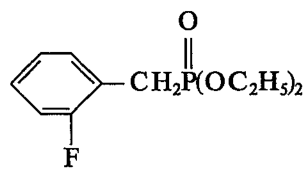 | 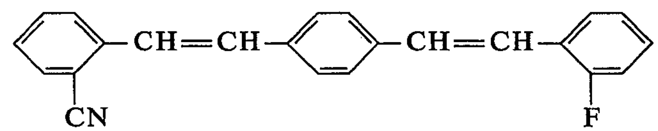 |
| 13 | desgl. | 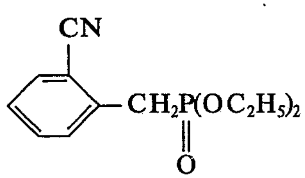 | 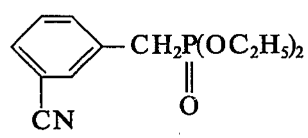 | 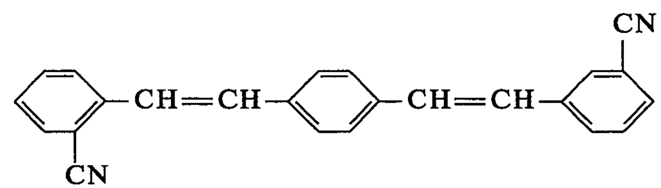 |

Beispiel 14

Man verfährt wie in Beispiel 1 und setzt 14,4 Teile Terephthalaldehyd nacheinander mit 25 Teilen o-Cyanbenzylphosphonester, 15,2 Teilen m-Cyanbenzylphosphonester und 10 Teilen p-Cyanbenzylphosphonester um. Man erhält ein Aufhellergemisch von

60% 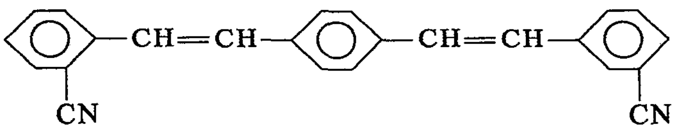

und

40% 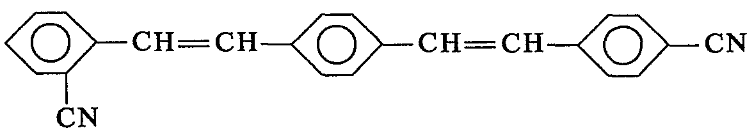

das ebenfalls hervorragend zur Polyesteraufhellung geeignet ist.

**Patentansprüche**

1. Verfahren zur Herstellung von unsymmetrischen Verbindungen oder Verbindungsgemischen der allgemeinen Formel

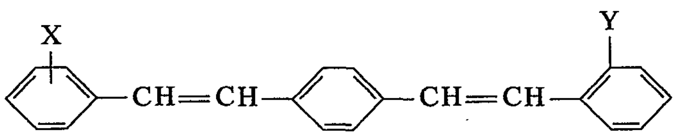

(I)

in der
X und Y unabhängig voneinander Wasserstoff, Fluor, Chlor, Cyan, $C_1$- bis $C_{10}$-Alkoxycarbonyl, gegebenenfalls substituiertes Carbamoyl oder Sulfamoyl, Sulfonsäurearylester, $C_1$- bis $C_{10}$-Alkylsulfonyl oder Phenylsulfonyl bedeuten, dadurch gekennzeichnet, daß man Terephthalaldehyd mit Verbindungen der allgemeinen Formeln IIa und IIb

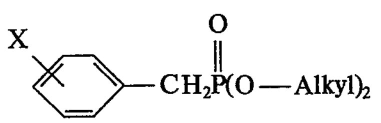

(IIa)

und

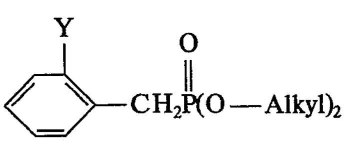

(IIb)

nacheinander in einem Ester als Lösungsmittel mit Alkali umsetzt, in dem das Monokondensationsprodukt ausfällt, wobei »Alkyl« in den Formeln $C_1$- bis $C_4$-Alkyl bedeutet.

2. Verfahren gemäß Anspruch 1, dadurch gekennzeichnet, daß man als Lösungsmittel Methylglykolacetat verwendet.

3. Verfahren gemäß Anspruch 1, dadurch gekennzeichnet, daß man Verbindungen oder Verbindungsgemische herstellt, bei denen X und Y Cyan sind.

**Claims**

1. A process for the preparation of asymmetrical compounds of the general formula

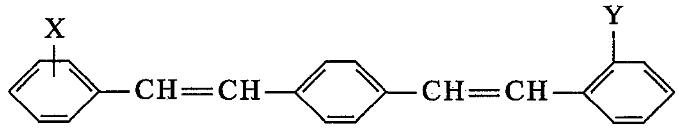

(I)

where

X and Y independently of one another are hydrogen, fluorine, chlorine, cyano, $C_1$-$C_{10}$-alkoxycarbonyl, unsubstituted or substituted carbamyl or sulfamyl, a sulfonic acid aryl ester group, $C_1$-$C_{10}$-alkylsulfonyl or phenylsulfonyl, or of mixtures of such compounds, wherein terephthalaldehyde is reacted successively with a compound of the general formula IIa

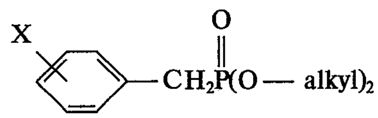

(IIa)

and a compound of the general formula IIb

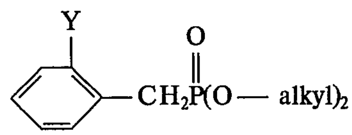

(IIb)

in the presence of an alkali, in an ester as solvent from which the monocondensation product precipitates, alkyl in the formulae denoting $C_1-C_4$-alkyl.

2. A process as claimed in claim 1, wherein methylglycol acetate is used as the solvent.

3. A process as claimed in claim 1, wherein a compound or mixture of compounds in which X and Y are cyano is prepared.

**Revendications**

1. Procédé pour la préparation de composés ou mélanges de composés dissymétriques de formule générale

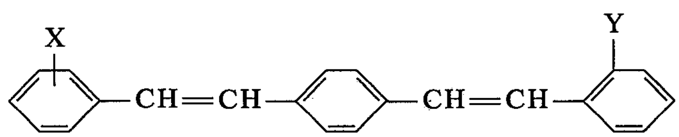

(I)

dans laquelle

X et Y représentent, indépendamment l'un de l'autre, un atome d'hydrogène, de fluor, de chlore, un groupe cyano, alcoxy($C_1-C_{10}$)-carbonyle, carbamoyle ou sulfamoyle éventuellement substitué, ester arylique d'acide sulfonique, alcoyl($C_1-C_{10}$)-sulfonyle ou phénylsulfonyle, caractérisé en ce qu'on fait réagir l'aldéhyde téréphtalique successivement avec des composés de formules générales IIa et IIb

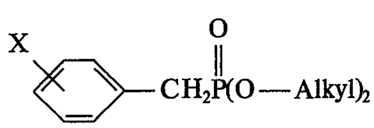

(IIa)

et

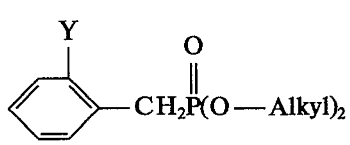

(IIb)

dans un ester servant de solvant avec un alcali, dans lequel le produit de monocondensation précipite, »alkyl« signifiant dans ces formules un alcoyle en $C_1$ à $C_4$.

2. Procédé selon la revendication 1, caractérisé en ce qu'on utilise comme solvant l'acétate de méthylglycol.

3. Procédé selon la revendication 1, caractérisé en ce que l'on prépare des composés ou mélanges de composés dans lesquels X et Y sont des groupes cyano.